# EUROPÄISCHE PATENTSCHRIFT

(11) **EP 2 364 354 B1**
(45) Veröffentlichungstag und Bekanntmachung des Hinweises auf die Patenterteilung: **26.09.2012**
(21) Anmeldenummer: 09764184.9
(22) Anmeldetag: 24.11.2009
(51) Int. Cl.: C12H 1/16, C12C 7/14, C12C 7/24, C12H 1/02, G01N 33/14

(54) **VERFAHREN ZUM ERMITTELN DER FILTRIERBARKEIT VON BIER**
METHOD FOR DETERMINING THE FILTERABILITY OF BEER
PROCÉDÉ DE DÉTERMINATION DE LA FILTRABILITÉ D'UNE BIÈRE

(30) Priorität: 04.12.2008 DE 102008060446
(43) Veröffentlichungstag der Anmeldung: 14.09.2011
(73) Patentinhaber: Krones AG, 93073 Neutraubling (DE)
(72) Erfinder: SCHNEID, Ralph, 85356 Freising (DE)
(74) Vertreter: Grünecker, Kinkeldey, Stockmair & Schwanhäusser
(86) Internationale Anmeldenummer: PCT/EP2009/008364
(87) Internationale Veröffentlichungsnummer: WO 2010/063392

(56) Entgegenhaltungen:
- WO-A1-2007/054146
- DE-A1- 2 443 735
- DE-A1- 19 831 946
- US-A1- 2003 168 413

## Beschreibung

Die Erfindung betrifft ein Verfahren zum Ermitteln der Filtrierbarkeit von Bier.

Da Bier nicht nur einen einwandfreien Geruch, Geschmack und Schaum aufweisen muss, sondern auch Glanzfeinheit verlangt wird, muss das Bier künstlich geklärt, d.h. filtriert werden. Dieser Vorgang bringt den Vorteil mit sich, dass hierbei nicht nur Trübungsbildner, wie Eiweiß, Gerbstoffverbindungen und Hopfenharze zurückbehalten werden, sondern auch Hefen und eventuell Bakterien. Der Filter ist in der Regel auf dem Abfüllweg zwischen Gär- /Lagertank und Füller angeordnet. Verschiedenen Biere weisen z.B. aufgrund unterschiedlicher Rohstoffeigenschaften oder unterschiedlicher Prozessführung eine unterschiedliche Filtrierbarkeit auf, d.h., dass ein unterschiedlich großer Anteil an Bestandteilen pro Zeiteinheit und Filterfläche aus dem Bier oder der Würze gefiltert werden kann und damit auch die Standzeit der entsprechenden Filter unterschiedlich hoch ist. Ein Problem bei der Filtration besteht nämlich darin, dass es beispielsweise im Fall der Kieselgurfiltration zu einem raschen Druckanstieg an der Filterfläche und damit zu einer geringeren Filterstandzeit kommt.

Bislang ist man davon ausgegangen, dass Biere mit einer ähnlichen Zusammensetzung, z. B. einem ähnlichen Betaglucan-Anteil, Einweißgehalt etc. und einer ähnlichen Viskosität eine ähnliche Filtrierbarkeit aufweisen. Interne Versuche haben jedoch folgendes gezeigt:

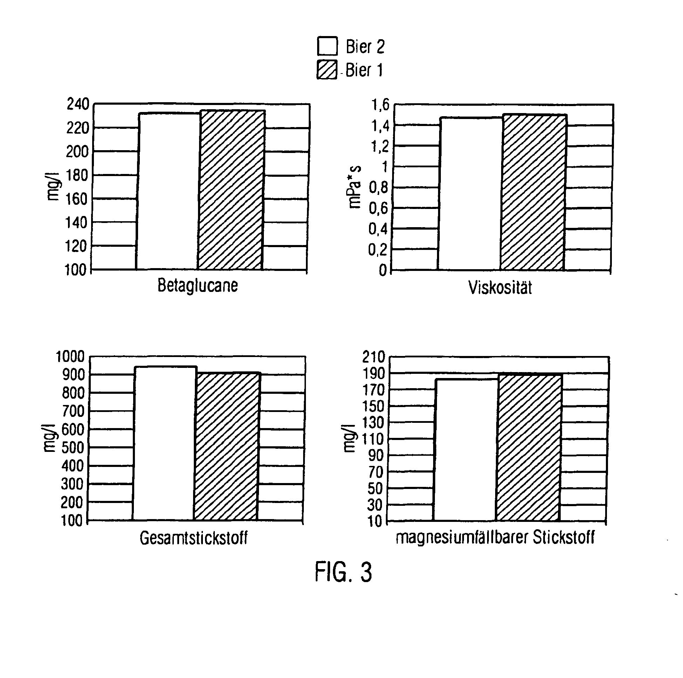

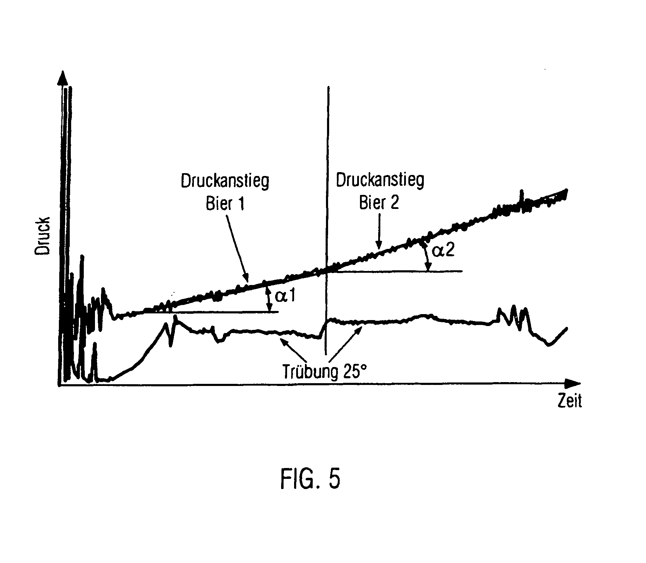

Fig. 3 zeigt als Beispiel den Anteil von Betaglucan, Gesamtstickstoff und Magnesium fällbaren Stickstoff, sowie die Viskosität für ein Standardbier, sowie ein Bier, das mit Hilfe eines Maischgefäßes hergestellt wurde, bei dem eine Vibrationseinheit (oder mehrere) vorgesehen ist, die die Maische in Schwingung versetzt. Ein solches Maischgefäß ist beispielsweise in der DE 10026723 A1 erläutert. Obwohl die mit und ohne Vibrationseinheit(en) hergestellten Biere, wie in Fig. 3 gezeigt ist, im wesentlichen die gleiche Zusammensetzung aufweisen, weisen diese doch eine unterschiedliche Filtrierbarkeit auf, wie deutlich aus Fig. 5 hervorgeht.

In der Fig. 5 ist der Anstieg der Druckdifferenz bei einem Kieselgurkerzenfilter zu sehen. Die linke Hälfte zeigt den Druckanstieg des Bieres 1 (α 1), die rechte Hälfte ein klassisch gebrautes Bier 2 (α 2). Es ist deutlich zu erkennen, dass der Druckanstieg in der linken Hälfte flacher verläuft (α 1 < α 2). Somit kann beim Bier 1 bis zum Erreichen der maximal zulässigen Druckdifferenz am Filter eine größere Menge Bier filtriert werden. Das heißt aber, dass mit den bisherigen Analyseverfahren, d.h. beispielsweise Bestimmung des Betaglucan-Anteils, der Viskosität etc. keine zuverlässige Aussage über die Filtrierharkeit gemacht werden kann.

Der vorliegenden Erfindung liegt daher die Aufgabe zugrunde ein Verfahren zum Ermitteln der Filtrierbarkeit von Bier bereitzustellen.

Erfindungsgemäß wird dieses Aufgabe durch die Merkmale des Anspruchs 1 gelöst.

Gemäß der vorliegenden Erfindung wird also in einem Schritt a) eine Würze- oder Bierprobe entnommen. Um eine Aussage über die Partikelgrößenverteilung einzelner Stoffe oder Stoffgruppen zu machen wird die Probe in einem Schritt b) an mehreren Filtern mit verschiedener Porengröße gefiltert. Dadurch, dass die Probe hintereinander an mehreren Filtern filtriert wird, verbleiben unterschiedliche Fraktionen auf den Filterflächen, so dass die Größe der Partikel auf der Filterfläche zuverlässig und einfach bestimmt werden kann. Hier muss natürlich nicht die gesamte Filterfläche untersucht werden. Es reicht, wenn ein repräsentativer Ausschnitt der Filterfläche untersucht wird. Durch qualitative Analyse der Partikel unterschiedlicher Größe auf den entsprechenden Filterflächen können die Partikel dann, bestimmten im Bier enthaltenden Stoffen oder Stoffgruppen, zugeordnet werden.

Vorteilhafterweise wird Schritt c) und d) für alle Fraktionen durchgeführt. Es ist jedoch auch möglich, diese Schritte nur für einige ausgewählte Fraktionen durchzuführen. Schritt d) kann auch vor Schritt c) durchgeführt werden.

Somit kann die Größe der Partikel unterschiedlicher Stoffe oder Stoffgruppen einzelner Fraktionen bestimmt werden. Diese ist ein Maß für die Filtrierbarkeit des Bieres. Somit kann mit Hilfe des erfindungsgemäßen Verfahrens eine Prognose über die Filtrierbarkeit erstellt werden, d.h. über die Größenordnung des Anteils an unerwünschten Bierinhaltsstoffen der pro Zeiteinheit und Filterfläche aus dem Bier oder der Würze gefiltert werden kann und welche Standzeit des Filters in etwa zu erwarten ist. Gemäß einer bevorzugten Ausführungsform wird die Partikelgrößenverteilung eines Stoffes oder einer Stoffgruppe in der Probe bestimmt. Dazu kann dann auch die Anzahl der Partikel einer bestimmten Größe eines bestimmten Stoffes oder einer Stoffgruppe ermittelt werden.

Bislang war nicht bekannt, dass die Größe unterschiedlicher Stoffe oder Stoffgruppen bzw. deren Partikelgrößenverteilung einen wesentlichen Einfluss auf die Filtrierbarkeit hat. Vorteilhafterweise wird als Filter ein Labor- oder Probefilter, insbesondere ein Membranfilter verwendet. Unter Laborfilter versteht man einen Filter, der deutlich kleinere Filterflächen als ein Filter zur Bierfiltration aufweist. Mit Hilfe eines solchen Membranfilters kann das Verfahren besonders einfach durchgeführt werden, da die Probe lediglich durch eine bestimmte Membran geleitet werden muss, und diese dann einfach entnommen werden kann.

Es ist sehr vorteilhaft, wenn der Technologe eine Prognose über die Filtrierbarkeit machen kann, da er dann in Abhängigkeit der Größe der Partikel unterschiedlicher Stoffgruppen einzelner Fraktionen Parameter des Bierherstellungsprozesses und/oder des Filterprozesses einstellen kann.

Es ist besonders vorteilhaft, wenn in Schritt c) die Größe der Partikel mittels Rasterelektronenmikroskop (REM) ermittelt wird. Die Größe der Partikel kann so einfach vermessen werden. Unter Größe der Partikel versteht man insbesondere deren maximale Länge, Fläche oder Durchmesser.

Besonders vorteilhaft ist es, wenn die qualitative Analyse der Partikel in Schritt d) mittels EDX-Verfahren (energiedispersive Röntgenanalyse) durchgeführt wird. Dazu kann nämlich eine Mikrobereichsanalytik im Mikro- und/oder Nanometerbereich vorgenommen werden, und das durch das Rasterelektronenmikroskop erzeugte Bild für die Identifizierung der Partikel verwendet werden. Somit kann die Zusammensetzung der Rückstände auf den Filterflächen, d.h. der Partikel auf einfache Art und Weise bestimmt werden. Da ein Großteil der Stoffe bzw. Stoffgruppen im Bier organisch ist, d.h. auf Kohlenstoff basiert, kann die Unterscheidung der Partikel anhand von bestimmten Indikatoren erfolgen (z.B. Stickstofffür Proteine). Mit diesem Verfahren lässt sich die Oberflächentopografie sehr gut darstellen.

Vorteilhafterweise werden zwei bis sechs Filter, insbesondere aber drei bis fünf Filter mit jeweils unterschiedlichen Porenweiten nacheinander verwendet. Die Porenweiten liegen dabei in einem Bereich von 0,1 bis 20 µm, vorzugsweise 0,45 bis 10 µm. Somit können auf den Filterflächen die Partikel unterschiedlicher Größe zurückbleiben und gut vermessen werden.

Die Stoffgruppen umfassen beispielsweise Betaglucan, Betaglucangele, Polyphenole (Gerbstoffe), höhere Dextrine, proteinische Substanzen. Die vorliegende Erfindung wird nachfolgend unter Zuhilfenahme der folgenden Figuren näher erläutert.

Vorteilhafterweise wird die Filterschicht vor der EDX-Analyse beschichtet, insbesondere mit einer Metallschicht oder einer Kohlenstoffschicht. So können die Partikel zur Analyse auf der Filterfläche fixiert werden. Die Bildqualität kann dadurch verbessert werden.

Es ist vorteilhaft die Probe vor der Filtration zu verdünnen. So kann das Ausbilden einer Deckschicht auf den Filterflächen vermieden werden, und die einzelnen Partikel zuverlässig untersucht werden.

Die qualitative Analyse in Schritt d) kann auch optisch durch Vergleichen des Aussehens der Partikel erfolgen.

Die vorliegende Erfindung wird nachfolgend unter Bezugnahme der folgenden Figuren näher erläutert.
- Fig. 1: zeigt in schematischer Darstellung die Filtrierbarkeit in Abhängigkeit der Partikelgröße unterschiedlicher Stoffgruppen für zwei verschiedene Biere.
- Fig. 2: zeigt einen Ablaufplan eines Ausführungsbeispiels der vorliegenden Erfindung.
- Fig. 3: zeigt Eigenschaften von zwei unterschiedlichen Bieren.
- Fig. 4A: zeigt eine Rasterelektronenmikroskopieaufnahme eines Filterflächenausschnittes.
- Fig. 4B: zeigt eine qualitative Analyse der Elementzusammensetzung, der in Fig. 4A gezeigten Aufnahme.
- Fig. 5: zeigt den unterschiedlichen Druckanstieg bei der Filtration von zwei unterschiedlichen Bieren.
- Fig. 6: zeigt einen Anordnung von mehreren Filtern gemäß der vorliegenden Erfindung
- Fig. 7: zeigt die Partikeldichte auf der Filterfläche bei unterschiedlichen Verdünnungen.
- Fig.8: zeigt die Oberfläche einer vergrößerten, planen Filterfläche, die besonders geeignet für die vorliegende Erfindung ist.

Im Folgenden wird ein mögliches Ausführungsbeispiel der vorliegenden Erfindung zum Ermitteln der Filtrierbarkeit von Bier näher erläutert.

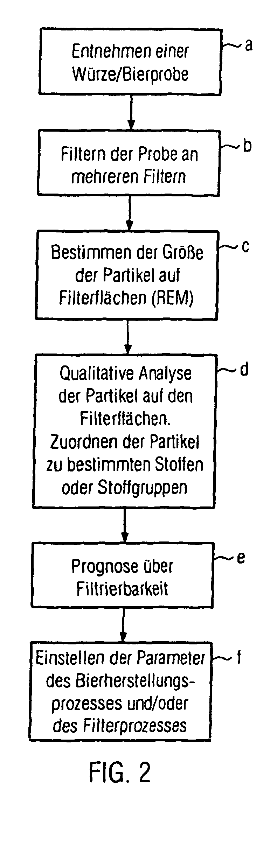

Wie insbesondere dem Ablaufdiagramm in Fig. 2 zu entnehmen ist, wird zunächst eine Würze oder Bierprobe des zu filtrierenden Bieres aus der Fertigung entnommen (a). Die Bierprobe umfasst dabei beispielsweise ein Volumen von 100 bis 200 ml. Im Labor wird dann vorzugsweise eine geeignete Verdünnung von z.B. 1:4 bis 1:10 z.B. mit Wasser hergestellt. Eine Verdünnung ist deshalb vorteilhaft, weil dann eine Partikeldeckschicht auf den jeweiligen Filterflächen vermieden werden kann, so dass die einzelnen Partikel auf der Filterfläche untersucht werden können.

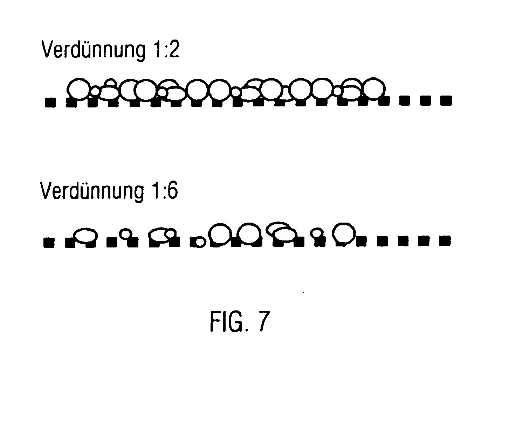

Fig. 7 zeigt beispielsweise die Partikeldichte auf einer Filterfläche bei einer Verdünnung 1:2. Hier ist die Partikeldichte so groß, dass eine Untersuchung der einzelnen Partikel nur schwer möglich ist. Bei einer Verdünnung von 1:6 ist die Partikeldichte geringer, so dass sowohl eine quantitative (Zählen der Partikel , Größenbestimmung) als auch eine qualitative Analyse gut möglich ist.

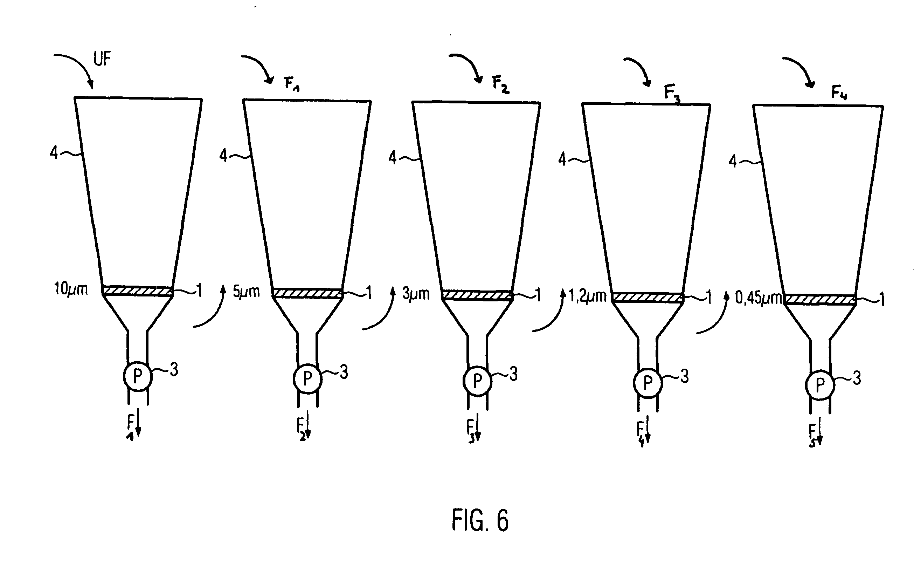

Als Ausgangsvolumen für das Verfahren steht dann beispielsweise eine verdünnte Probe mit einem Volumen von 100 ml bereit. Diese Probe wird dann über mehrere Laborfilter, hier Membranfilter verschiedener Porengröße nacheinander filtriert (b). Nacheinander bedeutet, wie aus Fig. 6 hervorgeht, dass das Filtrat F eines ersten Filters dem nachfolgenden Filter als Unfiltrat UF zugeführt wird, bis die Probe alle Filter passiert hat. Die Porenweite der unterschiedlichen Filter liegt in einem Bereich von 0,1 bis 20 µm, vorzugsweise 0,45 bis 10 µm. Bei diesem konkreten Ausführungsbeispiel werden, wie insbesondere aus der Fig 6 hervorgeht, beispielsweise Membranfilter mit Membranen einer Porengröße von 10 µm, 5 µm, 3 µm, 1,2 µm und 0,45 µm eingesetzt. Die Filter mit großer Porenweite, insbesondere der erste und zweite Filter dienen zur Abtrennung der Hefen. Die Fraktionierung sollte mit mindestens zwei bis sechs, vorzugsweise mit mindestens drei bis fünf Membranfiltern durchgeführt werden.

Durch die Fraktionierung können die Partikel in der Probe, insbesondere der verdünnten Probe, in verschiedene Größenbereiche aufgetrennt werden

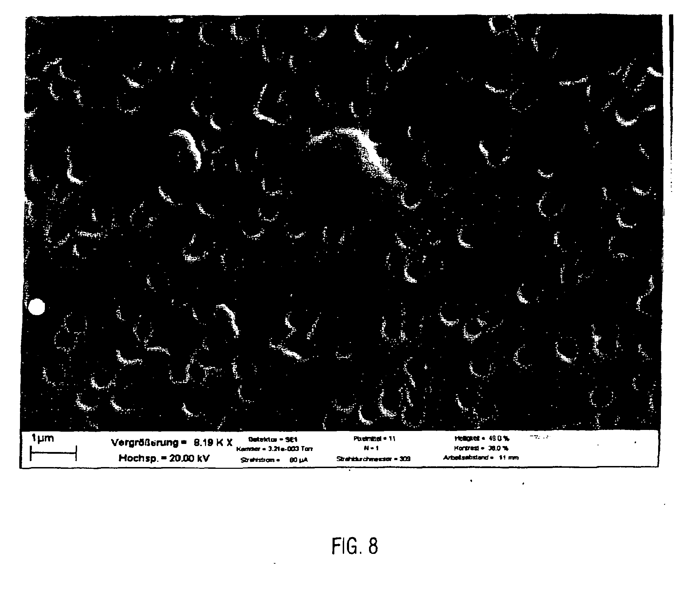

Die Filterfläche 1, d.h. die Membran, ist austauschbar im Filter angeordnet. Das Unfiltrat kann, wie in Fig. 6 gezeigt wird, in einen Unfiltratraum 4, hier Trichter, gefüllt werden, und mit Hilfe einer Pumpe P (z.B. Wasserstrahlpumpe) durch die Filtermembran gezogen werden Somit bleiben auf den einzelnen Filterflächen, d.h. auf den einzelnen Membranen Partikel unterschiedlicher Größe zurück. Die Größe der Filterfläche 1 liegt beispielsweise bei 10 - 30 cm².Als Filter eignet sich besonders gut ein Filter mit einer planen Oberfläche, d.h. dass die Oberfläche nicht strauchartig oder zerklüftet ist. Eine solche Filterfläche ist in Fig. 8 in 8,19K X Vergrößerung gezeigt. Dies bringt den Vorteil mit sich, dass die gefilterten Partikel alle mehr oder weniger auf einer Ebene liegen. Dies ist für die nachfolgende qualitative Untersuchung vorteilhaft. Als sehr geeignet hat sich ein Polycarbonatfilter, insbesondere ein stickstofffreier Filter mit Wabenstruktur erwiesen.

In einem nächsten Schritt (c) soll nun die Größe der Partikel auf der Filterfläche bestimmt werden. Dabei kann entweder die gesamte Filterfläche oder aber ein repräsentativer Ausschnitt der Filterfläche untersucht werden. Dazu wird die Filterfläche aus dem Filter ausgebaut. Die untersuchte Filterfläche liegt vorzugsweise in einem Bereich von 0,5 - 4 cm², z.B ein Quadrat mit einer Kantenlänge von 1 cm.

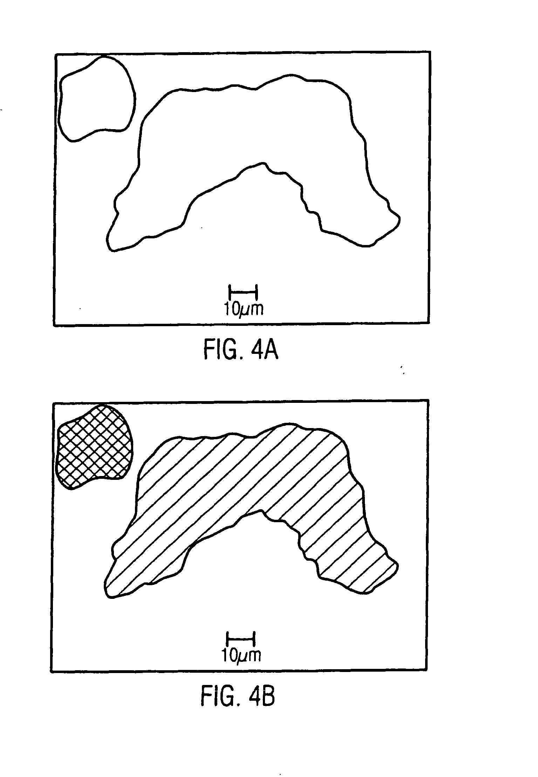

Besonders vorteilhaft ist es, wenn die Größe der Partikel mit Hilfe eines Rasterelektronenmikroskops vermessen wird. Unter Größe der Partikel versteht man die maximale Länge, Fläche oder Durchmesser. Fig. 4A zeigt beispielsweise einen Ausschnitt eines Rasterelektronenmikroskopbildes, das zwei Partikel auf der Filterfläche zeigt.

Schließlich erfolgt eine qualitative Analyse der Partikel (c) auf den Filterflächen. Vorteilhafterweise erfolgt diese qualitative Analyse mit Hilfe eines EDX-Verfahrens (energiedispersive Röntgenanalyse). Zur qualitativen EDX Analyse ist es vorteilhaft wenn die Filterfläche mit den Partikeln dünn beschichtet wird, d.h. z.B. eine Metallschicht ( z.B. Gold, Platin) oder eine Kohlenstoffschicht insbesondere durch Sputtern darauf abgeschieden wird. Dies ist hilfreich die Partikel zu fixieren, da ansonsten der Elektronenstrahl die Partikel wegschießen könnte. Durch die Schicht über der Filterfläche verbessert sich außerdem die Bildqualität. Wenn die Beschichtung vor dem Ausmessen der Partikelgröße erfolgt, kann die Schichtdicke beim Ausmessen berücksichtigt und abgezogen werden.

Bei dem EDX-Verfahren trifft ein Elektronenstrahl auf die Probenoberfläche. Durch Wechselwirkung zwischen Elektronen und Probe werden Röntgenstrahlen freigesetzt. Die Röntgenstrahlen werden detektiert, wobei elementspezifische Banden zur Elementanalyse verwendet werden. Da ein Großteil der Inhaltsstoffe des Bieres organisch ist, d.h. auf Kohlenstoff basiert, muss die Unterscheidung der Partikel anhand von Indikatoren erfolgen (z.B. Stickstoff für Proteine). Bei den anderen Stoffen oder Stoffgruppen muss der Anteil der Elemente ausgewertet werden. Wie aus Abbildung 4B hervorgeht, wird das Rasterelektronenmikroskopiebild bzw. die Oberfläche der Probe dann in unterschiedlichen Darstellungsweisen (z.B. Farben), die den entsprechenden detektierten Elementen entsprechen, dargestellt. Vorteilhafterweise wird ein REM/EDX Kombigerät verwendet.

Es ist genauso gut möglich, die Reihenfolge der Schritte c und d zu tauschen, so dass zunächst die qualitative Analyse der Partikel erfolgt und dann die Größe der entsprechenden Partikel bestimmt wird.

Auf jeden Fall können die vermessenen Partikel dann den in der Würze oder dem Bier enthaltenen Stoffen bzw. Stoffgruppen zugeordnet werden (d). Solche Stoffgruppen sind beispielsweise Betaglucan, Betaglucangele, Polyphenole, höhere Dextrine, proteinische Substanzen. Die relativ großen Hefen bleiben hauptsächlich bereits auf dem ersten, gröbsten Filter zurück, so dass für die Hefen keine weitere Analyse bezüglich Größe oder Zusammensetzung erfolgt. Der erste Filter muss daher in der Regel nicht zur weiteren Analyse herangezogen werden und dient somit lediglich als Vorfilter, damit die Partikel nachfolgender Fraktionen besser erfasst werden können. Somit kann ein Unterschied der Partikelgrößen der einzelnen Stoffe oder Stoffgruppen in den einzelnen Fraktionen ermittelt werden. Es müssen auch nicht alle Partikel auf der Filterfläche vermessen werden, sondern es reicht eine repräsentative Menge.

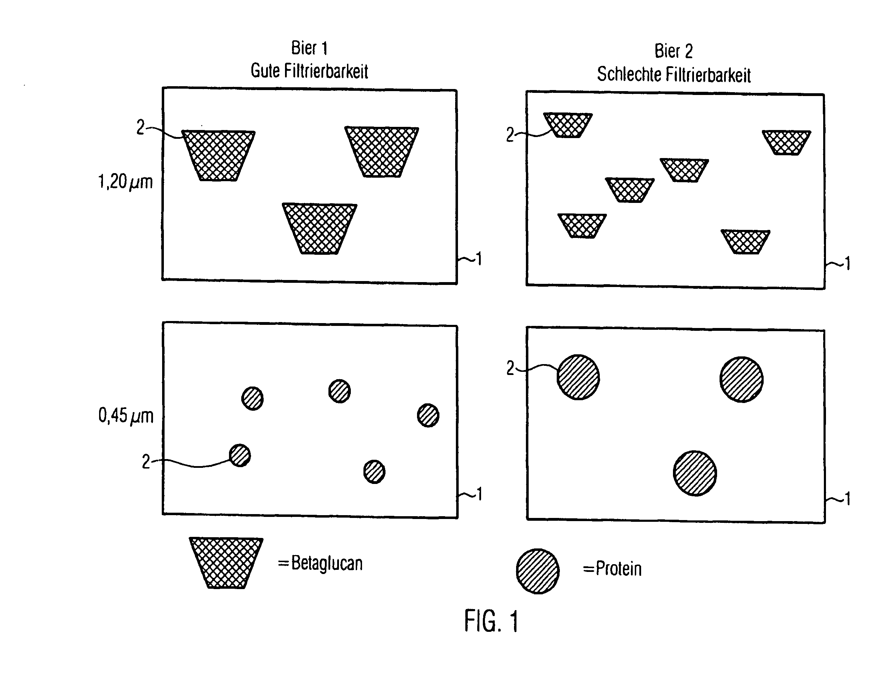

Fig. 1 zeigt die Ergebnisse der Schritte a bis d für ein Bier 1, sowie die Ergebnisse für ein Bier 2. Bier 1 ist ein Standardbier, während Bier 2 beim Maischen einem Vibrationsprozess, wie in der Beschreibungseinleitung erläutert, unterzogen wurde. Hier sind nur die Filterflächenausschnitte der Filter mit einer Porenweite von 1,2 µm und 0,45 µm dargestellt. In Fig. 1 werden ausschließlich zwei Fraktionen dargestellt. Es können auch alle Fraktionen in die Beurteilung mit eingehen.

Wie Fig. 1 zu entnehmen ist, weist das Bier 1 bei der Porengröße von 1,2 µm große Betaglukanpartikel auf. Die Betaglucanpartikel weisen hier beispielsweise eine Größe in einem Bereich von 5 bis 10 µm auf. Bei der Fraktion der Filterfläche mit einer Porengröße von 0,45 µm beinhaltet Bier 1 relativ kleine Proteinpartikel, die beispielsweise eine Größe von 0,5-1,0 µm aufweisen.

Im Gegensatz dazu zeigt das Bier 2 bei der 1,2 µm Fraktion kleinere Betaglucanpartikel, die beispielsweise eine Größe von 2-3 µm aufweisen. Im Gegensatz dazu finden sich auf der Filterfläche der 0,45 µm Fraktion relativ große Proteinpartikel, die beispielsweise eine Größe 1-1,2 µm aufweisen.

Es ist auch möglich einen Durchschnittswert der Größe von Partikeln einer bestimmten Stoffgruppe einer bestimmten Fraktion zu bilden.

Es können jedoch auch im wesentlichen alle Partikel eines Stoffes oder einer Stoffgruppe auf der Filterfläche bzw. des bestimmten Abschnitts der Filterfläche gezählt werden, so dass dann die Partikelgrößenverteilung des Stoffes oder der Stoffgruppe in der gesamten Probe bestimmt werden kann.

Die Ergebnisse werden dann mit Vergleichsergebnissen verglichen, die in Vergleichsversuchen ermittelt wurden. In den entsprechenden Vergleichsversuchen wurde die Filtrierbarkeit bei unterschiedlichen Größen der Partikel bestimmter Stoffe oder Stoffgruppen bestimmt.

So weist beispielsweise Bier 1 mit sehr großen Betaglucan-Partikeln und kleinen Proteinpartikeln eine bessere Filtrierbarkeit auf, als Bier 2, das kleinere Betaglucan-Partikel aufweist, jedoch größere Proteinpartikel. Das bedeutet, dass bei Bier 1 ein größerer Anteil an Bierinhaltsstoffen pro Zeiteinheit und Filterfläche aus dem Bier oder der Würze gefiltert werden kann und eine größere Standzeit des Filters zu erwarten ist als bei Bier 2. Dies liegt z.B. daran, dass die größeren Betaglucan-Partikel die Bierfilterfläche locker halten und damit die kleinen Proteinpartikel den Filter besser passieren können.

Das heißt, dass eine Prognose über die Filtrierbarkeit gemacht werden kann (e).

Dem Technologen ist somit die Möglichkeit gegeben, gezielt in den Bierherstellungsprozess und/oder den Filterprozess in Abhängigkeit der ermittelten Größe der Partikel unterschiedlicher Stoffgruppen einzugreifen. (f)

Bei einer Anschwemmfiltration kann dann eine entsprechend gröbere oder feinere Kieselgurmischung bei der Dosage, so wie ein angepasster Celluloseanteil bei der Voranschwemmung geplant werden. Das heißt, dass beispielsweise bei Bier 2, das eine schlechtere Filtrierbarkeit aufweist eine Dosage mit gröberem Kieselguranteil und höherem Celluloseanteil bei der Voranschwemmung angesetzt werden kann.

Bei der Kieselgur, d.h. filterhilfsmittelfreien Filtration kann zumindest die Produktionsplanung genauer kalkuliert werden.

Eine Vorhersage der Filtrierbarkeit sollte aus einer Probe der Anstellwürze bzw. noch früher aus einer Probe der Kongresswürze gemacht werden. Weiterhin kann als Probe auch das endvergorene Bier, zum Beispiel aus der Anstellwürze beziehungsweise der Kongresswürze verwendet werden. Darüber hinaus kann als Probe zum Beispiel ebenfalls das endvergorene Bier aus dem Produktionsprozess der Bierherstellung dienen. Hier wurden ais Beispiel die unterschiedlich hergestellten Biere 1 und 2 angeführt. Der Vollständigkeit halber sollte jedoch noch angemerkt werde, dass sich unterschiedliche Filtrierbarkeiten auch bei gleichem Herstellungsverfahren aufgrund unterschiedlicher Rohstoffeigenschaften ergeben.

Die qualitative Analyse bzw. die Zuordnung zu verschiedenen Stoffen oder Stoffgruppen kann durch die zuvor beschriebene instrumentelle Analytik und/oder optisch erfolgen; d.h. auch aufgrund der speziellen Form oder des Aussehens der Partikel erfolgen (wie beispielsweise porös, kompakt, glatt, kugelförmig, strukturiert, gelartig) und durch Vergleich der speziellen Form bzw. Aussehens der im Mikroskop (REM) dargestellten Partikeln mit der zuvor im Mikroskop (REM) ermittelten Form von bestimmten Stoffen oder Stoffgruppen einzelner Fraktionen.

Der vollkommen neue Ansatz, die Filtrierbarkeit in Abhängigkeit der Größe unterschiedlicher Stoffe bzw. Stoffgruppen zu beurteilen, ermöglicht letztendlich ein verbessertes Filtrationsverfahren, bei dem die Filter eine längere Standzeit haben

## Patentansprüche

1. Verfahren insbesondere zum Ermitteln der Filtrierbarkeit von Bier mit folgenden Schritten:
a) Entnehmen einer Würze- oder Bierprobe,
b) Filtern der Probe an mehreren Filtern mit verschiedener Porengröße,
c) Bestimmen der Größe der Partikel auf Filterflächen der Filter,
d) qualitative Analyse der Partikel auf den Filterflächen und Zuordnung der vermessenen Partikel zu bestimmten im Bier oder der Würze enthaltenen Stoffen oder Stoffgruppen,
wobei aus der Größe der Partikel unterschiedlicher Stoffe oder Stoffgruppen einzelner Fraktionen Aussagen für die Filtrierbarkeit abgeleitet werden können.

2. Verfahren nach Anspruch 1, **dadurch gekennzeichnet, dass** die Partikelgrößenverteilung eines Stoffes oder einer Stoffgruppe in der Probe bestimmt wird.

3. Verfahren nach Anspruch 1 oder 2, **dadurch gekennzeichnet, dass** das Verfahren weiter folgenden Schritt umfasst:
e) Einstellen von Parametern des Bierherstellungsprozesses und/oder des Filterprozesses in Abhängigkeit der Größe der Partikel unterschiedlicher Stoffgruppen einzelner Fraktionen.

4. Verfahren nach Anspruch 1, **dadurch gekennzeichnet, dass** als Filter ein Laborfilter, insbesondere ein Membranfilter für die Vorhersage der Filtrierbarkeit verwendet wird.

5. Verfahren nach mindestens einem der Ansprüche 1 bis 4, **dadurch gekennzeichnet, dass** in Schritt c) die Größe der Partikel mittels REM ermittelt wird.

6. Verfahren nach mindestens einem der Ansprüche 1 bis 5, **dadurch gekennzeichnet, dass** die qualitative Analyse der Partikel in Schritt d) mittels EDX-Verfahren bestimmt wird.

7. Verfahren nach mindestens einem der Ansprüche 1 bis 6, **dadurch gekennzeichnet, dass** zwei bis sechs Filter, insbesondere drei bis fünf Filter mit jeweils unterschiedlicher Porenweite nacheinander verwendet werden, wobei die Porenweite in einem Bereich von 0,1 bis 20 µm vorzugsweise 0,45 bis 10 µm liegt.

8. Verfahren nach mindestens einem der Ansprüche 1 bis 7, **dadurch gekennzeichnet, dass** die Stoffgruppen Betaglucan, Betaglucangele, Polyphenole, höhere Dextrine und proteinische Substanzen umfassen.

9. Verfahren nach mindestens einem der Ansprüche 1 bis 8, **dadurch gekennzeichnet, dass** auf der Filterfläche vor Schritt d) eine Schicht, insbesondere eine Kohlenstoff - oder Metallschicht abgeschieden wird.

10. Verfahren nach mindestens einem der Ansprüche 1 bis 9, **dadurch gekennzeichnet, dass** die Probe vor der Filtration verdünnt wird.

11. Verfahren nach mindestens einem der Ansprüche 1-10, **dadurch gekennzeichnet, dass** die qualitative Analyse in Schritt d) optisch erfolgt durch Vergleich des Aussehens der Partikel auf der Filterfläche mit dem Aussehen von Partikeln bestimmter Stoffe oder Stoffgruppen.

## Claims

1. Method in particular for determining the filterability of beer, comprising the following steps:
a) taking a wort or beer sample,
b) filtering the sample on a plurality of filters of varying pore size,
c) determining the size of the particles on the filter surfaces of the filters,
d) qualitative analysis of the particles on the filter surfaces and assigning the measured particles to certain substances or groups of substances present in the beer or wort,
wherein evidence on the filterability can be obtained from the size of the particles of different substances or groups of substances of individual fractions.

2. Method according to claim 1, **characterised in that** the particle size distribution of a substance or a group of substances is determined in the sample.

3. Method according to claim 1 or 2, **characterised in that** the method furthermore comprises the following step:
e) adjusting parameters of the beer manufacturing process and/or the filtering process depending on the size of the particles of different groups of substances of individual fractions.

4. Method according to claim 1, **characterised in that** as a filter, a laboratory filter, in particular a membrane filter, is used for the prediction of filterability.

5. Method according to at least one of claims 1 to 4, **characterised in that** in step c), the size of the particles is determined by means of REM.

6. Method according at least to one of claims 1 to 5, **characterised in that** the qualitative analysis of the particles in step d) is determined by means of an EDX method.

7. Method according to at least one of claims 1 to 6, **characterised in that** two to six filters, in particular three to five filters, each with different pore widths, are used successively, where the pore width is within a range of 0.1 to 20 µm, preferably 0.45 to 10 µm.

8. Method according to at least one of claims 1 to 7, **characterised in that** the groups of substances comprise beta glucan, beta glucan gels, polyphenols, higher dextrins and proteinaceous substances.

9. Method according to at least one of claims 1 to 8, **characterised in that** a coating, in particular a carbon or metal coating, is deposited on the filter surface before step d).

10. Method according to at least one of claims 1 to 9, **characterised in that** the sample is diluted before filtration.

11. Method according to at least one of claims 1-10, **characterised in that** the qualitative analysis in step d) is effected optically by comparing the appearance of the particles on the filter surface with the appearance of particles of certain substances or groups of substances.

## Revendications

1. Procédé en particulier pour déterminer la filtrabilité de la bière, avec les étapes suivantes :
a) prélèvement d'un échantillon de moût ou de bière,
b) filtration de l'échantillon sur plusieurs filtres de tailles de pores différentes,
c) définition de la taille des particules sur les surfaces filtrantes des filtres,
d) analyse qualitative des particules sur les surfaces filtrantes, et affectation des particules mesurées à des substances ou groupes de substances définis qui sont contenus dans la bière ou dans le moût,
étant précisé que des informations pour la filtrabilité peuvent être déduites de la taille des particules de différentes substances ou groupes de substances de fractions individuelles.

2. Procédé selon la revendication 1, **caractérisé en ce que** la répartition de taille de particules d'une substance ou d'un groupe de substances dans l'échantillon est définie.

3. Procédé selon la revendication 1 ou 2, **caractérisé en ce que** le procédé comprend par ailleurs l'étape suivante :
e) réglage de paramètres du processus de fabrication de bière et/ou du processus de filtration en fonction de la taille des particules de différents groupes de substances de fractions individuelles.

4. Procédé selon la revendication 1, **caractérisé en ce qu'**on utilise comme filtre un filtre de laboratoire, en particulier un filtre à membrane, pour prévoir la filtrabilité.

5. Procédé selon l'une au moins des revendications 1 à 4, **caractérisé en ce que** lors de l'étape c), la taille des particules est déterminée par REM (microscope électronique à balayage).

6. Procédé selon l'une au moins des revendications 1 à 5, **caractérisé en ce que** l'analyse qualitative des particules est définie lors de l'étape d) à l'aide d'un procédé EDX (analyse par dispersion d'énergie)

7. Procédé selon l'une au moins des revendications 1 à 6, **caractérisé en ce que** deux à six filtres, en particulier trois à cinq filtres de largeurs de pores différentes sont utilisés successivement, étant précisé que la largeur de pores est située dans une plage de 0,1 à 20 µm, de préférence de 0,45 à 10 µm.

8. Procédé selon l'une au moins des revendications 1 à 7, **caractérisé en ce que** les groupes de substances comprennent le bêtaglucane, les gels de bêtaglucane, les polyphénols, les dextrines supérieures et les substances protéiniques.

9. Procédé selon l'une au moins des revendications 1 à 8, **caractérisé en ce qu'**une couche, en particulier une couche de carbone ou de métal, est séparée sur la surface filtrante avant l'étape d).

10. Procédé selon l'une au moins des revendications 1 à 9, **caractérisé en ce que** l'échantillon est dilué avant la filtration.

11. Procédé selon l'une au moins des revendications 1 à 10, **caractérisé en ce que** l'analyse qualitative se fait par voie optique, lors de l'étape d), grâce à une comparaison de l'aspect des particules sur la surface filtrante avec l'aspect de particules de substances ou de groupes de substances définis.
